(19) Europäisches Patentamt
European Patent Office
Office européen des brevets

(11) Veröffentlichungsnummer: **0 004 641**
**A2**

(12) # EUROPÄISCHE PATENTANMELDUNG

(21) Anmeldenummer: **79100942.6**

(22) Anmeldetag: **29.03.79**

(51) Int. Cl.²: **C 07 C 51/56**
C 07 C 63/06, C 07 C 53/26
C 07 C 61/08

(30) Priorität: **11.04.78 DE 2815541**

(43) Veröffentlichungstag der Anmeldung:
**17.10.79 Patentblatt 79/21**

(84) Benannte Vertragsstaaten:
**BE CH DE FR GB IT NL**

(71) Anmelder: **BAYER Aktiengesellschaft**
**Zentralbereich Patente,Marken und Lizenzen Bayerwerk**
**D-5090 Leverkusen 1(DE)**

(72) Erfinder: **Lenthe, Manfred, Dr.**
**Michaelshöhe 40**
**D-5074 Odenthal(DE)**

(72) Erfinder: **Findeisen, Kurt, Dr.**
**In der Follmühle 10**
**D-5074 Odenthal(DE)**

(72) Erfinder: **Dankert, Gerhard, Dr.**
**Arthur-Hantzsch-Strasse 44**
**D-5000 Köln 80(DE)**

(72) Erfinder: **Grah, Gerd**
**Goethestrasse 2**
**D-5657 Haan(DE)**

(54) **Verfahren zur Herstellung von Carbonsäureanhydriden.**

(57) Die Erfindung betrifft ein Verfahren zur abluft- und abwasserfreien Herstellung von Carbonsäureanhydriden wie Benzoesäureanhydrid und Pivalinsäureanhydrid aus den entsprechenden Carbonsäuren und Essigsäureanhydrid. Die Umsetzung wird erfindungsgemäß in einer bei Normaldruck oder vermindertem Druck kontinuierlich oder diskontinuierlich betriebenen Reaktions-Destillations-Apparatur (vgl. Fig. 1) unter gleichzeitiger Trennung der Reaktionsprodukte Carbonsäureanhydrid und Essigsäure durchgeführt.

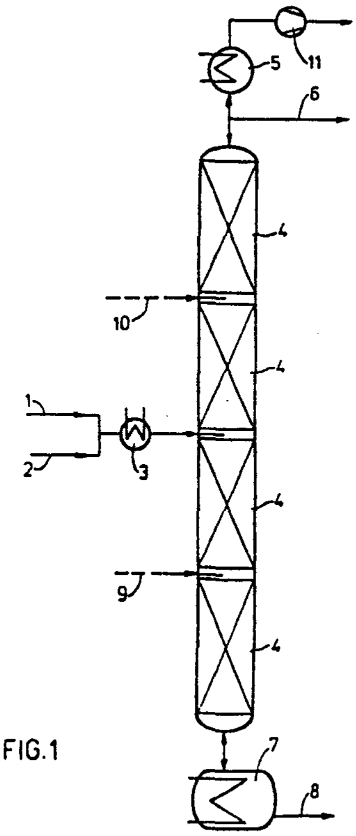


FIG.1

EP 0 004 641 A2

Croydon Printing Company Ltd.

BAYER AKTIENGESELLSCHAFT
Zentralbereich
Patente, Marken und Lizenzen

5090 Leverkusen, Bayerwerk
Bi-by-Bs
IVa/ZP

## Verfahren zur Herstellung von Carbonsäureanhydriden

Die vorliegende Erfindung betrifft ein Verfahren zur abluft- und abwasserfreien Herstellung von bestimmten, relativ hoch siedenden Carbonsäureanhydriden, wie Benzoesäureanhydrid und Pivalinsäureanhydrid, aus den entsprechenden Carbonsäuren und Essigsäureanhydrid in technischem Maßstab, in einer kontinuierlich oder diskontinuierlich betriebenen Destillations-Apparatur unter gleichzeitiger Abtrennung des Leichtsieders Essigsäure. Carbonsäureanhydride sind wertvolle Zwischenprodukte für zahlreiche Synthesen der organischen Chemie.

Die Gewinnung von Benzoesäureanhydrid aus Benzoesäure und Essigsäureanhydrid als Wasserakzeptor gemäß Reaktionsschema (1)

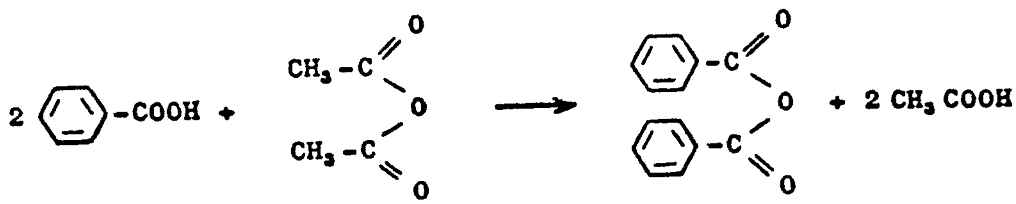

(1)

ist eine lange bekannte Reaktion und gelingt mit Ausbeuten von maximal 81 % (Ber. 42, S. 3483 (1909); vgl. auch Houben-Weyl, Methoden der Organischen Chemie, Band VIII, S. 476 (1952); ferner Org. Synth. Coll. Vol. I, S. 91). Nachteilig bei diesem bekannten Verfahren sind der sehr hohe Einsatzüberschuß an Essigsäureanhydrid, der zur Erzielung der genannten Ausbeute mindestens 100 % betragen muß, die hohe Reaktionszeit von z.B. 6 Stunden und die aufgrund der Reaktionsbedingungen erforderliche aufwendige Produktionsanlage mit hohen Energie- und Personalkosten.

Andere bekannte Verfahren gehen aus von Benzoylchlorid oder Benzotrichlorid (vgl. die oben zit. Literaturstellen in Houben-Weyl und Org. Synth.). Aber auch bei diesen Verfahren ist ein hoher technischer Aufwand erforderlich, und es fallen salzhaltige Abwässer an, die eine technische Nutzung unwirtschaftlich machen.

Eine direkte thermische Dehydratisierung ist bei den hier interessierenden Carbonsäuren nicht durchführbar.

Es wurde nun gefunden, daß man nach (1) die Carbonsäureanhydride der Formel

$$R - CO - O - CO - R \qquad (I)$$

in welcher

R für gegebenenfalls substituiertes Phenyl, Cyclohexyl oder tert.-Butyl steht,

in praktisch quantitativer Ausbeute, hoher Reinheit und in extrem kurzen Reaktionszeiten erhält, wenn man ein - vorzugsweise stöchiometrisches - Gemisch aus einer Carbonsäure der Formel

$$R - COOH \qquad (II)$$

in welcher

R die oben angegebene Bedeutung hat,

und Essigsäureanhydrid in eine unter Normaldruck oder verminderten Druck kontinuierlich betriebene Destillationskolonne einführt. Man erhält ein Kopfprodukt mit einem Gehalt von 98 - 100 % Essigsäure (Rest: Essigsäureanhydrid) und ein ebenso hochreines farbloses Carbonsäureanhydrid im Sumpf; die Ausbeuten an Carbonsäureanhydrid (I) erreichen 100 %. Es treten hierbei weder Abluft noch Abwasser auf.

Die durch Formel (II) repräsentierten Ausgangsprodukte sind Benzoesäure, substituierte Benzoesäuren, Hexahydrobenzoesäure (= Cyclohexancarbonsäure) und Pivalinsäure (= Trimethylessigsäure). Die Benzoesäure kann beispielsweise durch folgende bevorzugte Reste substituiert sein:

Methyl, Äthyl, Halogen (insbesondere Chlor), Trifluormethyl, Methoxy, Äthoxy, Methoxycarbonyl, Äthoxycarbonyl, Cyano oder Nitro. Besonders bevorzugt ist Benzoesäure, ferner Pivalinsäure.

Das erfindungsgemäße Verfahren kann im Druckbereich zwischen 1000 und 10 mbar durchgeführt werden. Wegen der relativ hohen Siedepunkte der gebildeten Carbonsäureanhydride (I), (z.B. 360°C für Benzoesäureanhydrid) betreibt man die Kolonne zweckmäßigerweise bei vermindertem Druck, vorzugsweise im Bereich zwischen 300 und 10 mbar, insbesondere im Bereich zwischen 200 und 20 mbar; in Abhängigkeit vom gewählten Betriebsdruck stellen sich die Temperaturen in der Kolonne ein.

Grundsätzlich ist auch eine Verfahrensweise unter erhöhtem Druck denkbar. Die dabei auftretenden hohen Sumpftemperaturen lassen dies jedoch als weniger zweckmäßig erscheinen.

Die besten Ausbeuten, bezogen auf die eingesetzte Carbonsäure (II), lassen sich bei stöchiometrischem Einsatzverhältnis oder mäßigem Essigsäureanhydridüberschuß erzielen. Bei Anhydridmangel kann zwar ein Essigsäureanhydrid-freies Destillat gewonnen werden, dafür enthält das Produkt jedoch nichtumgesetzte Carbonsäure (II). In Frage kommt ein Einsatzverhältnis von etwa 0,5 bis 0,75 Mol, vorzugsweise von 0,5 Mol, Essigsäureanhydrid pro Mol Carbonsäure (II).

Üblicherweise wird eine Destillationskolonne mit einem einzigen Zulauf betrieben, über den das vorgewärmte Gemisch zugeführt wird. (Vgl. Fig. 1) Bei dieser Betriebsart werden auch hier die angegebenen, sehr guten Ergebnisse erzielt.

Eine weitere Verbesserung des Verfahrens läßt sich jedoch dadurch erreichen, daß die Zuläufe für Essigsäureanhydrid und Carbonsäure (II) getrennt werden, und zwar so, daß Essigsäureanhydrid im unteren Teil, die Carbonsäure dagegen im oberen Teil der Kolonne zugeführt wird (vgl. ebenfalls Fig. 1). Mit dieser Variante kann entweder die Stufenzahl der Destillationskolonne vermindert oder die Kolonne bei einem kleineren Rücklauf-Destillat-Verhältnis betrieben werden, wodurch eine höhere Belastbarkeit der Apparatur bewirkt wird. In beiden Fällen werden Abschreibungskosten eingespart.

Mit gleich hohen Ausbeuten läßt sich die Reaktion auch diskontinuierlich durchführen, indem ein Rührkessel mit einem gut trennenden Destillationsaufsatz versehen wird. Das Carbonsäure-Essigsäureanhydrid-Gemisch wird bei Normaldruck oder vermindertem Druck zum Sieden erhitzt, und das Rücklaufverhältnis wird so eingestellt, daß am Kopf reine Essigsäure entnommen wird. Arbeitet man bei vermindertem Druck, so gilt der gleiche Druckbereich wie oben für die kontinuierliche Verfahrensweise angegeben. Auch für das Einsatzverhältnis der Ausgangsprodukte gelten die obigen Angaben.

Die diskontinuierliche Verfahrenweise ist ebenso umweltfreundlich wie die oben beschriebene kontinuierliche, da ebenfalls keine Abluft und kein Abwasser auftreten.

Gegenüber den bekannten Verfahren zeichnet sich die neue Technik durch weitere wesentliche Vorteile aus, welche direkten Einfluß auf die Wirtschaftlichkeit des Verfahrens nehmen:

Durch die Möglichkeit, die Komponenten exakt stöchiometrisch einzusetzen, wird die Trennung von Essigsäure- Essigsäureanhydrid-Gemischen vermieden, die sonst mit zusätzlichen Investitions- und vor allem hohen Energiekosten durchgeführt werden müßte. Die hier anfallende Essigsäure, die maximal nur ca. 2 % Essigsäureanhydrid enthält, kann ohne weitere Reinigungsmaßnahmen einem Abnehmer zugeführt werden. Die zur Produktion benötigte Anlage besteht im wesentlichen nur noch aus einem einzigen Apparat - im Falle der bevorzugten kontinuierlichen Verfahrensweise aus einer Reaktions-Destillations-Kolonne (vgl. Fig. 1), im Falle der diskontinuierlichen Verfahrensweise aus der oben beschriebenen, etwas modifizierten Reaktions-Destillations-Apparatur - welcher, verglichen mit den für die bekannten Verfahren benötigten aufwendigen Anlagen, geringere Abschreibungen und Personalkosten erfordert.

Carbonsäureanhydride werden häufig verwendet als Ersatzchemikalien für Säurechloride mit einer vergleichbaren chemischen Reaktionsfähigkeit, aber ohne die bei der Verwendung von Säurechloriden auftretende Korrosion zu verursachen. Auch die Verwendung von Carbonsäureanhydriden als Zwischenprodukte für Wirkstoffe des Pflanzenschutzes ist möglich: z.B. können die Carbonsäureanhydride (I) zur Herstellung von 1,2,4-Triazin-5-onen eingesetzt werden, welche hervorragende herbizide Eigenschaften besitzen. So läßt sich beispielsweise, ausgehend von Benzoesäureanhydrid, das bekannte, herbizid wirksame 3-Methyl-4-amino-6-phenyl-1,2,4-triazin-5-on (common name: Metamitron) herstellen (vgl. DE-OS 2 224 161, ferner DE-OS 2 614 240 und DE-OS 2 614 242).

In analoger Weise können aus Cyclohexancarbonsäureanhydrid und Pivalinsäureanhydrid z.B. die herbizid wirksamen Verbindungen 3-tert.-Butyl-4-amino-6-cyclohexyl-1,2,4-triazin-5-on bzw. 3-Äthyl-4-amino-6-tert.-butyl-1,2,4-triazin-5-on (vgl. US-PS 3 874 914) hergestellt werden.

Das erfindungsgemäße Verfahren wird durch die nachfolgenden Herstellungsbeispiele veranschaulicht.

Herstellungsbeispiele

Bei den Versuchen zur Durchführung des erfindungsgemäßen Verfahrens in einer kontinuierlich betriebenen Destillationskolonne wurde eine Apparatur benutzt, wie sie in der Fig. 1 dargestellt ist.

Die Reaktanden Essigsäureanhydrid (1) und Carbonsäure (II) (z.B. geschmolzene Benzoesäure) (2) werden in einem Vorheizer (3) vermischt, auf 150°C geheizt und in die Kolonne gegeben (4), die in vier Schüsse unterteilt ist, deren Packungshöhe 850 mm bei einem Durchmesser von 50 mm beträgt. Die Schüsse sind mit einer elektrischen Schutzheizung versehen, die ein Temperaturgefälle zwischen Säuleninnerem und Mantel von 15°C erzeugt.

Das Destillat (6) wird am Kopf der Kolonne (5) über einen Rücklaufteiler entnommen, das Produkt, Carbonsäureanhydrid (I) (z.B. Benzoesäureanhydrid) wird im Sumpfablauf (7)⟶(8) gewonnen.

Der Betriebsdruck der Anlage wird am Kopf gemessen und mittels Pumpe und Fremdluftventil konstant gehalten (11).

Beispiel 1

In die beschriebene Anlage (Fig. 1) werden 2665 g/h Benzoesäure und 1120 g/h Essigsäureanhydrid über einen gemeinsamen Vorwärmer eingegeben. Der Betriebsdruck beträgt am

Kopf der Kolonne 100 mbar, die Sumpftemperatur 188°C und die Kopftemperatur 57,4°C bei einem Rücklaufverhältnis von 3:1. Nach Erreichen des stationären Zustandes werden vom Sumpf 2510 g/h Produkt entnommen mit einem Gehalt von 98,0 Benzoesäureanhydrid. Die Ausbeute beträgt 99,7 %. Das Destillat besteht aus reiner Essigsäure und fällt mit 1280 g/h an.

Beispiel 2

In die Anlage gemäß Beispiel 1 werden bei einem Druck von 50 mbar 2665 g/h Benzoesäure und 1139 g/h Essigsäureanhydrid über einen gemeinsamen Vorwärmer eingefahren. Bei einer Kopftemperatur von 44,3°C werden 1033 g/h Destillat mit einem Gehalt von 100 % Essigsäure und bei der Sumpftemperatur von 193°C ein Produktstrom von 2500 g/h mit einem Benzoesäureanhydridgehalt von 98,7 %, Rest Benzoesäure, erhalten. Das Rücklaufverhältnis beträgt (wie in Beispiel 1) 3:1. Die Ausbeute beträgt 100 %.

Beispiel 3

Für das folgende Beispiel wurde die Kolonne so verändert, wie es in der Fig. 1 durch die gestrichelten Linien angedeutet wird. Der gemeinsame Zulauf der beiden Komponenten wird ersetzt durch zwei einzelne Zuläufe. Essigsäureanhydrid

(9) wird oberhalb des unteren Viertels der Kolonne, Benzoesäure (10) dagegen unterhalb des oberen Vierteils zugeführt. Beide Komponenten werden in nicht eingezeichneten Wärmeaustauschern vorgeheizt: Essigsäureanhydrid auf 50°C, Benzoesäure auf 150°C.

In diese Apparatur werden bei einem Druck von 50 mbar 2665 g/h Benzoesäure und 1132 g/h Essigsäureanhydrid eingebracht. Die Sumpftemperatur wird auf 191°C eingestellt, und bei einem Rücklaufverhältnis von 2:1 wird eine Kopftemperatur von 44,5°C erreicht. Es werden 1190 g/h Destillat erhalten mit 100 % Essigsäure und 2480 g/h Sumpfablauf mit 99,2 % Benzoesäureanhydrid entsprechend einer Ausbeute von 99,7 %.

Beispiel 4

Dieses Beispiel dient zur Erläuterung der diskontinuierlichen Verfahrensführung. Ein 4-ltr.-Glaskolben wird mit einem Destillationsaufsatz versehen, der bei einem Durchmesser von 50 mm eine Packungshöhe von 850 mm hat. Die Einstellung des Rücklaufverhältnisses erfolgt über einen Dampfteiler.

1710 g Benzoesäure und 763 g Essigsäureanhydrid werden in dieser Apparatur bei 100 mbar zum Sieden erhitzt. Bei einem Rücklaufverhältnis von 5:1 wird der Druck gleichmäßig innerhalb von 3 Stunden auf 20 mbar gesenkt.

Die Sumpftemperatur steigt von anfänglich $100^{o}C$ auf zum Schluß $180^{o}C$. Es wird ein Sumpf von 1580 g mit einem Gehalt von 99 % Benzoesäureanhydrid und 887 g Destillat mit 91,5 % Essigsäure und 8,5 % Essigsäureanhydrid gewonnen. Die Ausbeute beträgt 98,6 %.

## Patentansprüche

1. Verfahren zur Herstellung von Carbonsäureanhydriden der Formel

$$R - CO - O - CO - R \qquad (I)$$

in welcher

R für gegebenenfalls durch Methyl, Äthyl, Halogen, Trifluormethyl, Methoxy, Äthoxy, Methoxycarbonyl, Äthoxycarbonyl, Cyano oder Nitro substituiertes Phenyl, Cyclohexyl oder tert.-Butyl steht,

aus den entsprechenden Carbonsäuren, R-COOH (II) und Essigsäureanhydrid, dadurch gekennzeichnet, daß die Reaktion in einer bei Normaldruck oder vermindertem Druck kontinuierlich oder diskontinuierlich betriebenen Reaktions-Destillations-Apparatur unter gleichzeitiger Trennung der Reaktionsprodukte Carbonsäureanhydrid (I) und Essigsäure durchgeführt wird.

2. Verfahren nach Anspruch 1, dadurch gekennzeichnet, daß die Reaktion in einer kontinuierlich betriebenen Reaktions-Destillations-Kolonne durchgeführt wird.

3. Verfahren nach Anspruch 1, dadurch gekennzeichnet, daß die Reaktion diskontinuierlich durchgeführt wird, in einer Apparatur bestehend aus einem Rührkessel mit gut trennendem Destillationsaufsatz.

4. Verfahren nach Anspruch 2, dadurch gekennzeichnet, daß die Ausgangsprodukte Carbonsäure (II) und Essigsäureanhydrid durch getrennte Zuläufe in die Kolonne eingeführt werden, und zwar dergestalt, daß die Carbonsäure (II) im oberen Teil der Kolonne, und Essigsäureanhydrid im unteren Teil der Kolonne, zugeführt werden.

5. Verfahren nach Anspruch 1, dadurch gekennzeichnet, daß die Reaktion im Druckbereich zwischen 1000 und 10 mbar, insbesondere im Bereich zwischen 200 und 20 mbar durchgeführt wird.

6. Verfahren nach Anspruch 1, dadurch gekennzeichnet, daß auf 1 Mol Carbonsäure (II) 0,5 bis 0,75 Mol, vorzugsweise 0,5 Mol, Essigsäureanhydrid eingesetzt werden.

7. Verfahren nach Anspruch 1, dadurch gekennzeichnet, daß als Carbonsäure (II) Benzoesäure eingesetzt wird.

8. Verfahren nach Anspruch 1, dadurch gekennzeichnet, daß als Carbonsäure (II) Cyclohexancarbonsäure eingesetzt wird.

9. Verfahren nach Anspruch 1, dadurch gekennzeichnet, daß als Carbonsäure (II) Pivalinsäure eingesetzt wird.

0004641

1/1

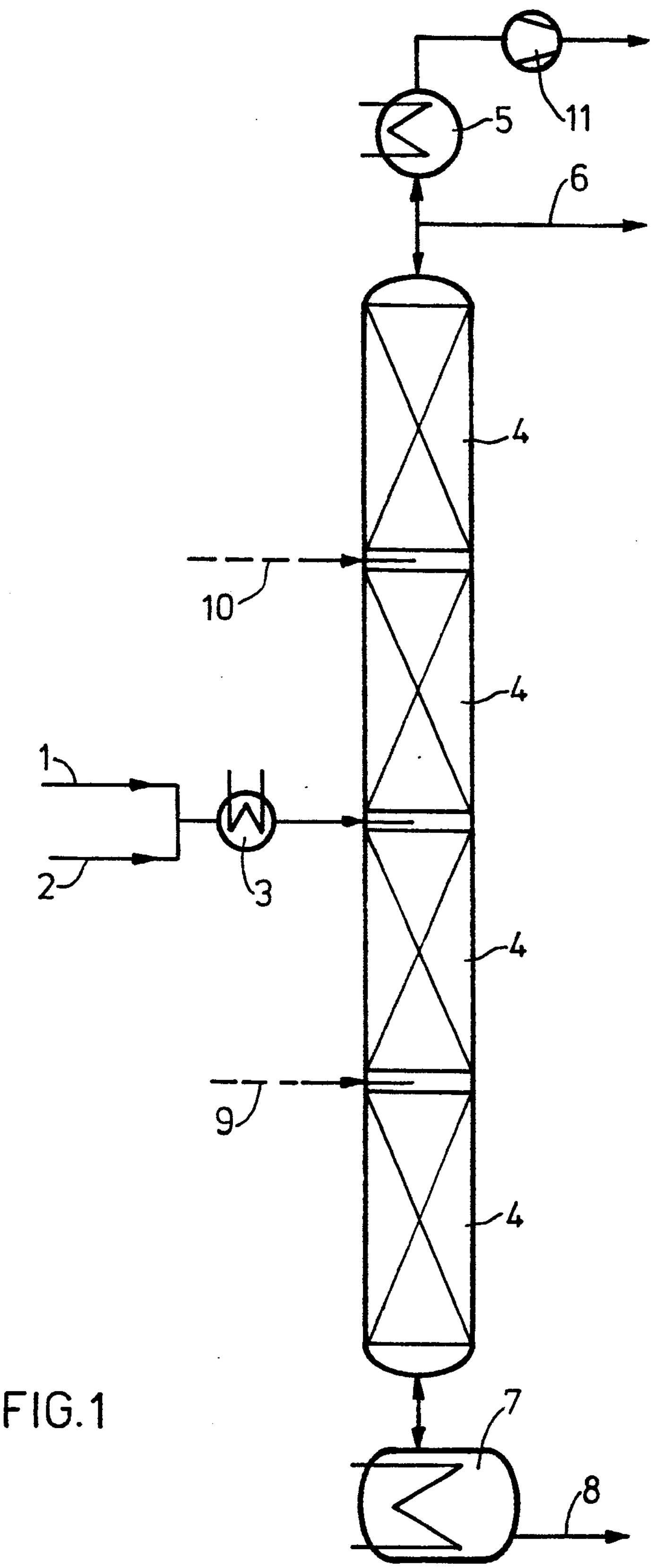


FIG.1